# EUROPEAN PATENT SPECIFICATION

(11) **EP 4 085 902 B1**
(45) Date of publication and mention of the grant of the patent: **30.04.2025**
(21) Application number: 20908545.5
(22) Date of filing: 30.12.2020
(51) Int. Cl.: A61K 9/20, C07D 215/24, A61P 35/00, A61K 9/28, A61K 31/47

(54) **PHARMACEUTICAL COMPOSITION CONTAINING NITROXOLINE, NITROXOLINE TABLET, PREPARATION METHOD THEREFOR AND USE THEREOF**
PHARMAZEUTISCHE ZUSAMMENSETZUNG MIT NITROXOLIN, NITROXOLINTABLETTE, VERFAHREN ZU IHRER HERSTELLUNG UND IHRE VERWENDUNG
COMPOSITION PHARMACEUTIQUE CONTENANT DE LA NITROXOLINE, COMPRIMÉ DE NITROXOLINE, PROCÉDÉ DE PRÉPARATION ASSOCIÉ ET UTILISATION CORRESPONDANTE

(30) Priority: 31.12.2019 CN 201911405993
(43) Date of publication of application: 09.11.2022
(73) Proprietor: Jiangsu Yahong Meditech Co., Ltd., Taizhou, Jiangsu 225316 (CN); Asieris Pharmaceuticals (Shanghai) Co., Ltd., Pudong, Shanghai 201203 (CN)
(72) Inventor: CHEN, Jie, Taizhou, Jiangsu 225316 (CN); SHEN, Shuai, Taizhou, Jiangsu 225316 (CN); LI, Haiyan, Taizhou, Jiangsu 225316 (CN); WU, Youbin, Taizhou, Jiangsu 225316 (CN); LIU, Jianghua, Taizhou, Jiangsu 225316 (CN); GUO, Yushen, Shanghai 201203 (CN)
(74) Representative: EP&C
(86) International application number: PCT/CN2020/141424
(87) International publication number: WO 2021/136381

(56) References cited:
- WO-A1-2014/145723
- WO-A1-2017/173278
- CN-A- 103 319 404
- CN-A- 103 446 069
- CN-A- 105 228 984
- CN-A- 109 106 715
- LAUWO J A K: "EFFECT OF PARTICLE SIZE AND EXCIPIENTS ON THE DISSOLUTION RATE OF METRONIDAZOLE FROM SOLID DOSAGE FORMS 2", JOURNAL DRUG DEVELOPMENT AND INDUSTRIAL PHARMACY, NEW YORK, NY, US, vol. 10, no. 7, 1 January 1984 (1984-01-01), pages 1085 - 1096, XP009171262, ISSN: 0363-9045, DOI: 10.3109/03639048409038307
- LIN, NING: "Dissolution Rate of Drugs", BIOPHARMACOLOGY AND PHARMACOKINETICS, 30 June 2017 (2017-06-30), CN, pages 27 - 30, XP009529204, ISBN: 978-7-5132-4102-1

## Description

### FIELD OF THE INVENTION

The present invention relates to a phamaceutical composition comprising nitroxoline, a nitroxoline tablet, a method for preparing the same, and a composition for use thereof

### BACKGROUND OF THE INVENTION

Nitroxoline, the chemical name of which is 5-nitro-8-hydroxyquinoline, was developed as an oral antibiotic drug in the 1960s. It was mainly used for urinary system infections, and had a safe history of use before being replaced due to discovery and use of new antibiotics.

In recent years, new studies have found that nitroxoline can simultaneously inhibit the methionine aminopeptidase MetAP2 and the silence information regulator 2-related enzyme SIRT1 in vascular endothelial cells, and exert a synergistic inhibitory effect on tumor angiogenesis, as well as an inhibitory effect on the proliferation of tumor cells. Therefore, nitroxoline can be developed to treat tumors including bladder cancer.

As discussed above, nitroxoline can be used for various clinical indications, such as urinary tract infection and bladder cancer. When oral administration is applied to treat diseases, different clinical indications have different requirements for drug dissolution rate. For example, the treatment of urinary tract infection involves a short-term administration that requires the drug to take effect as soon as possible, so a relatively fast drug dissolution rate is ideal to better achieve an effective anti-infective effect; while the treatment of bladder cancer involves a long-term administration that requires a narrow variation range of blood drug concentration, so a relatively moderate drug dissolution rate is ideal.

At present, there is no pharmaceutical composition containing nitroxoline with a moderate dissolution rate in the prior art. Therefore, the development of pharmaceutical composition containing nitroxoline with a moderate dissolution rate is an urgent technical problem to be solved at present.

### SUMMARY OF THE INVENTION

The technical problem to be solved by the present invention is to provide a phamaceutical composition containing nitroxoline, a nitroxoline tablet, a method for preparing the same, and a composition for use thereof, so as to overcome the defect that there is no pharmaceutical composition containing nitroxoline with a moderate dissolution rate in the prior art.

The inventors found in the research that nitroxoline has low solubility and slow dissolution in 0.1 mol/L hydrochloric acid, water and phosphate buffer (pH 6.8) at 37°C, and the properties of nitroxoline itself will have an adverse effect on obtaining a pharmaceutical composition containing nitroxoline with a moderate dissolution rate. After extensive research, the inventors found that pharmaceutical compositions containing nitroxoline with different dissolution rates can be obtained by controlling the particle size of active pharmaceutical ingredient (API). After further research, the inventors found that nitroxoline tablets with a moderate dissolution rate (the dissolution rate within 60 min is more than 75%) can be prepared when the particle size D₉₀ of nitroxoline is 10 to 100 µm. In addition, the resulting nitroxoline tablets have uniform content, narrow variation range in hardness, and good fluidity and compressibility.

The present invention solves the above technical problem through the following technical solutions:
The present invention provides a pharmaceutical composition, wherein the pharmaceutical composition according to the claims comprises an active pharmaceutical ingredient and a pharmaceutically acceptable carrier, the active pharmaceutical ingredient is nitroxoline or a pharmaceutically acceptable salt thereof, and the particle size D₉₀ of the active pharmaceutical ingredient is 10 to 100 µm.

The active pharmaceutical ingredient is nitroxoline.

In some preferred embodiments, the pharmaceutically acceptable salt of nitroxoline is a base addition salt formed by nitroxoline and an inorganic base or an organic base, an acid addition salt formed by nitroxoline and an inorganic acid or an organic acid, or a salt formed by nitroxoline and an alkaline or acidic amino acid, and preferably nitroxoline hydrochloride or lysine salt. The inorganic base is sodium hydroxide and/or sodium phosphate, the organic base is triethylamine and/or diethylamine, the inorganic acid is hydrochloric acid and/or phosphoric acid, the organic acid is tartaric acid and/or citric acid, the alkaline amino acid is lysine, and the acidic amino acid is glutamic acid.

In some preferred embodiments, the particle size D₉₀ of the active pharmaceutical ingredient is 10 to 70 µm, preferably 40 to 70 µm, for example 12.61 µm, 42.55 µm, 43.29 µm, 70.58 µm or 99.31 µm.

In some preferred embodiments, the particle size D₅₀ of the active pharmaceutical ingredient is 4 to 50 µm, preferably 4 to 40 µm or 4 to 35 µm, more preferably 10 to 40 µm, for example 4.58 µm, 10.88 µm, 11.02 µm, 14.95 µm or 33.60 µm.

In some preferred embodiments, the particle size D₁₀ of the active pharmaceutical ingredient is 0 to 10 µm, and is not 0, preferably 0.1 to 6 µm or 2 to 10 µm, and more preferably 2 to 6 µm, for example 0.12 µm, 3.44 µm, 3.67 µm, 3.99 µm or 5.66 µm.

According to the claims, the active pharmaceutical ingredient is present in an amount of 20 to 65 parts by weight or 20 to 60 parts by weight, preferably 25 to 60 parts by weight, more preferably 40 to 61 parts by weight, and further more preferably 56 to 61 parts by weight, for example 56.3 parts by weight, 60.1 parts by weight, 60.2 parts by weight or 60.4 parts by weight per 100 parts by weight of the pharmaceutical composition.

According to the claims, the carrier comprises a filler. The filler is one or more of starch, pregelatinized starch, partially pregelatinized starch, lactose, sucrose, mannitol, sorbitol, hydrous calcium phosphate, anhydrous calcium phosphate and microcrystalline cellulose, and more preferably one or more of starch, lactose and microcrystalline cellulose. The filler is preferably present in an amount of 30 to 300 parts by weight, more preferably 50 to 100 parts by weight, further more preferably 30 to 90 parts by weight, and still further more preferably 30 to 75 parts by weight per 100 parts by weight of the active pharmaceutical ingredient.

According to the claims, the carrier comprises a binder. The binder is one or more of hydroxypropyl cellulose, hydroxypropyl methylcellulose, polyvinylpyrrolidone and starch, more preferably one or more of polyvinylpyrrolidone, hydroxypropyl methylcellulose and starch, and further more preferably polyvinylpyrrolidone and/or starch. The binder is preferably present in an amount of 0 to 100 parts by weight, and not 0, more preferably 2 to 50 parts by weight, and further more preferably 30 to 50 parts by weight per 100 parts by weight of the active pharmaceutical ingredient.

According to the claims, the carrier comprises a disintegrant. The disintegrant is one or more of low-substituted hydroxypropyl cellulose. calcium carboxymethyl cellulose, sodium carboxymethyl starch, croscarmellose sodium and crospovidone, more preferably low-substituted hydroxypropyl cellulose and/or crospovidone, and further more preferably low-substituted hydroxypropyl cellulose. The disintegrant is preferably present in an amount of 0 to 100 parts by weight, and not 0, more preferably 1 to 25 parts by weight or 5 to 25 parts by weight, further more preferably 1 to 10 parts by weight, 2 to 10 parts by weight or 5 to 10 parts by weight, and still further more preferably 5 to 6 parts by weight per 100 parts by weight of the active pharmaceutical ingredient.

According to the claims, the carrier comprises a lubricant. The lubricant is one or more of magnesium stearate, sodium stearyl fumarate and sodium dodecyl sulfate, and more preferably sodium stearyl fumarate and/or sodium dodecyl sulfate. The lubricant is preferably present in an amount of 0.25 to 20 parts by weight, more preferably 0.5 to 5 parts by weight, and further more preferably 2.5 to 4 parts by weight per 100 parts by weight of the active pharmaceutical ingredient.

In some preferred embodiments, the carrier comprises a glidant. The glidant is preferably one or more of silica, talc and sodium dodecyl sulfate, more preferably silica and/or sodium dodecyl sulfate, and further more preferably sodium dodecyl sulfate. The glidant is preferably present in an amount of 0 to 20 parts by weight, and not 0, more preferably 0.5 to 10 parts by weight, and further more preferably 4 to 10 parts by weight per 100 parts by weight of the active pharmaceutical ingredient.

In some preferred embodiments, the carrier comprises a filler and a binder, wherein the filler and the binder are present in a total amount of 30 to 300 parts by weight, preferably 32 to 280 parts by weight, more preferably 32 to 140 parts by weight, and further more preferably 56.5 to 70 parts by weight per 100 parts by weight of the active pharmaceutical ingredient.

In some preferred embodiments, the carrier comprises a lubricant and a glidant, wherein the lubricant and the glidant are present in a total amount of 1 to 10 parts by weight, preferably 1 to 15 parts by weight, more preferably 2.5 to 10 parts by weight, and further more preferably 2.5 to 4 parts by weight per 100 parts by weight of the active pharmaceutical ingredient.

In some preferred embodiments, the pharmaceutical composition also comprises a coating agent.

The coating agent is preferably hydroxypropyl methylcellulose and/or polyvinyl alcohol, and more preferably hydroxypropyl methylcellulose.

The coating agent is preferably present in the pharmaceutical composition in an amount of 3% to 15% by mass, and more preferably 9% to 13% by mass relative to the mass of the uncoated pharmaceutical composition.

In some preferred embodiments, the pharmaceutical composition according to the claims comprises an active pharmaceutical ingredient and a pharmaceutically acceptable carrier, wherein the active pharmaceutical ingredient is nitroxoline or a pharmaceutically acceptable salt thereof; the particle size D₉₀ of the active pharmaceutical ingredient is 10 to 100 µm, the particle size D₅₀ of the active pharmaceutical ingredient is 4 to 35 µm, the particle size D₁₀ of the active pharmaceutical ingredient is 0.1 to 6 µm; the active pharmaceutical ingredient is present in an amount of 56 to 61 parts by weight per 100 parts by weight of the pharmaceutical composition;
the carrier comprises a filler and a binder, the filler is one or more of starch, lactose and microcrystalline cellulose, the binder is polyvinylpyrrolidone and/or starch; the filler and the binder are present in a total amount of 56.5 to 70 parts by weight per 100 parts by weight of the active pharmaceutical ingredient;
the carrier comprises a disintegrant, the disintegrant is low-substituted hydroxypropyl cellulose; the disintegrant is present in an amount of 5 to 6 parts by weight per 100 parts by weight of the active pharmaceutical ingredient;
the carrier comprises adjuvant I, the adjuvant I is sodium stearyl fumarate and/or sodium dodecyl sulfate; the adjuvant I is present in an amount of 2.5 to 4 parts by weight per 100 parts by weight of the active pharmaceutical ingredient;
the pharmaceutical composition also comprises a coating agent, the coating agent is hydroxypropyl methylcellulose, the coating agent is present in the pharmaceutical composition in an amount of 9% to 13% by mass relative to the mass of the uncoated pharmaceutical composition.

The present invention also provides a nitroxoline tablet, wherein the nitroxoline tablet is prepared from the aforementioned pharmaceutical composition.

The present invention also provides a method for preparing the nitroxoline tablet, wherein the pharmaceutical composition in the method is the aforementioned pharmaceutical composition, the pharmaceutical composition comprises adjuvant II, the adjuvant II is the aforementioned lubricant and/or the aforementioned glidant,
the method comprises the following steps of: subjecting the components in the pharmaceutical composition except adjuvant II to wet granulation, wet milling, drying and dry milling, adding adjuvant II, subjecting the resulting mixture to total mixing, and tableting to obtain the nitroxoline tablet.

In the above method, when the pharmaceutical composition comprises a coating agent, coating is performed after tableting.

The present invention also provides a composition for use of the aforementioned pharmaceutical composition and/or the aforementioned nitroxoline tablet for treating cancer. For example, the cancer can be bladder cancer.

The present invention provides a composition for use in a method for treating cancer, wherein the method comprises the following step of: administering to a subject a therapeutically effective amount of the aforementioned pharmaceutical composition and/or the aforementioned nitroxoline tablet.

The present invention also provides a nitroxoline solid tablet composition according to the claims, comprising an active pharmaceutical ingredient uniformly dispersed therein and a pharmaceutically acceptable carrier, wherein the active pharmaceutical ingredient is nitroxoline or a pharmaceutically acceptable salt thereof, the particle size D₉₀ of the active pharmaceutical ingredient is 10 to 100 µm, preferably 10 to 70 µm, and more preferably 40 to 70 µm.

In some preferred embodiments, the particle size D₅₀ of the active pharmaceutical ingredient is 4 to 50 µm, preferably 4 to 40 µm, and more preferably 10 to 40 µm.

In some preferred embodiments, the particle size D₁₀ of the active pharmaceutical ingredient is 0 to 10 µm, and is not 0, preferably 2 to 10 µm, and more preferably 2 to 6 µm.

In some preferred embodiments, the pharmaceutically acceptable salt of nitroxoline is a base addition salt formed by nitroxoline and an inorganic base or an organic base, an acid addition salt formed by nitroxoline and an inorganic acid or an organic acid, or a salt formed by nitroxoline and an alkaline or acidic amino acid, and preferably nitroxoline hydrochloride or lysine salt. The inorganic base is sodium hydroxide and/or sodium phosphate, the organic base is triethylamine and/or diethylamine, the inorganic acid is hydrochloric acid and/or phosphoric acid, the organic acid is tartaric acid and/or citric acid, the alkaline amino acid is lysine, and the acidic amino acid is glutamic acid.

According to the claims, the active pharmaceutical ingredient is present in an amount of 20 to 60 parts by weight, and preferably 25 to 60 parts by weight per 100 parts by weight of the pharmaceutical composition.

In some preferred embodiments, the pharmaceutically acceptable carrier is one or more of filler, disintegrant, binder, glidant, lubricant, colorant, pH adjuster, surfactant, stabilizer and fragrance; and preferably filler, disintegrant, binder, glidant and lubricant.

According to the claims, the filler is one or more of starch, pregelatinized starch, partially pregelatinized starch, lactose, sucrose, mannitol, sorbitol, hydrous calcium phosphate, anhydrous calcium phosphate and microcrystalline cellulose.

In some preferred embodiments, the filler is present in an amount of 30 to 300 parts by weight, preferably 50 to 100 parts by weight, more preferably 30 to 90 parts by weight, and further more preferably 30 to 75 parts by weight per 100 parts by weight of the active pharmaceutical ingredient.

According to the claims, the disintegrant is one or more of low-substituted hydroxypropyl cellulose, calcium carboxymethyl cellulose, sodium carboxymethyl starch, croscarmellose sodium and crospovidone.

In some preferred embodiments, the disintegrant is present in an amount of 0 to 100 parts by weight, and not 0, preferably 5 to 25 parts by weight, and more preferably 5 to 10 parts by weight per 100 parts by weight of the active pharmaceutical ingredient.

According to the claims, the binder is one or more of hydroxypropyl cellulose, hydroxypropyl methylcellulose, polyvinylpyrrolidone and starch.

In some preferred embodiments, the binder is present in an amount of 0 to 100 parts by weight, and not 0, preferably 2 to 50 parts by weight, and more preferably 30 to 50 parts by weight per 100 parts by weight of the active pharmaceutical ingredient.

According to the claims, the lubricant is one or more of magnesium stearate, sodium stearyl fumarate and sodium dodecyl sulfate.

In some preferred embodiments, the lubricant is present in an amount of 0.25 to 20 parts by weight, preferably 1 to 5 parts by weight, and more preferably 2.5 to 4 parts by weight per 100 parts by weight of the active pharmaceutical ingredient.

In some preferred embodiments, the glidant is one or more of silica, talc and sodium dodecyl sulfate.

In some preferred embodiments, the glidant is present in an amount of 0 to 20 parts by weight, and not 0, preferably 0.5 to 10 parts by weight, and more preferably 4 to 10 parts by weight per 100 parts by weight of the active pharmaceutical ingredient.

In some preferred embodiments, the hardness of the tablet is 6 to 20 kg.

In some preferred embodiments, the nitroxoline solid tablet composition further comprises a film coating material.

The film coating material can be hydroxypropyl methylcellulose and/or polyvinyl alcohol.

The weight gain of the film coating material is preferably 3% to 15% per 100 parts by weight of the active pharmaceutical ingredient.

The present invention also provides a nitroxoline tablet, comprising:

| | |
|---|---|
| nitroxoline | 100 parts by weight, |
| filler | 30 to 300 parts by weight, preferably 50 to 100 parts by weight, |
| disintegrant | 0 to 100 parts by weight, preferably 5 to 25 or 5 to 10 parts by weight, |
| binder | 0 to 100 parts by weight, preferably 5 to 25 or 2 to 50 parts by weight, |
| lubricant | 0.25 to 20 parts by weight, preferably 2.5 to 4 or 0.5 to 5 parts by weight, |
| glidant | 0 to 20 parts by weight, preferably 4 to 10 or 0.5 to 10 parts by weight, and |
| film coating powder | 3% to 15% weight gain, and |
| the particle size D₉₀ of nitroxoline is 10 to 100 µm, preferably 10 to 70 µm, and more preferably 40 to 70 µm. | |

In the aforementioned nitroxoline tablet, the types of the filler, disintegrant, binder, lubricant and glidant are as defined above.

The present invention also provides a method for preparing the aforementioned nitroxoline solid tablet, comprising the following steps of:
1) pulverizing the nitroxoline raw material and controlling the D₉₀ to be 10 to 100 µm;
2) weighing the pulverized particles obtained in step 1), filler, disintegrant, binder, glidant and lubricant according to the prescription;
3) formulating the binder;
4) mixing the pulverized particles, filler and disintegrant weighed in step 2) with the binder formulated in step 3), and subjecting the resulting mixture to wet granulation, wet milling, drying and dry milling;
5) adding glidant or lubricant to the dry granules obtained in step 4) to obtain the total mixed granules;
6) tableting the total mixed granules obtained in step 5) with a tableting machine;
7) optionally, film-coating the tablet obtained in step 6) with the film coating material to obtain the nitroxoline tablet.

In the aforementioned method, the nitroxoline raw material can be pulverized by a hammer mill, grinding mill or jet mill.

In the aforementioned method, the tableting can be direct tableting, wet granulation tableting or dry granulation tableting.

Disclosed, but not claimed, is a method for controlling the dissolution rate of the nitroxoline solid tablet, comprising the steps of pulverizing the active ingredient nitroxoline and controlling the particle size D₉₀ of nitroxoline to be 10 to 100 µm to prepare the nitroxoline solid tablet, wherein the cumulative dissolution rate of the nitroxoline solid tablet at 60 min is not less than 75%

Regarding the terms not defined herein, they have the meanings commonly understood by those skilled in the art. Regarding the terms defined herein, they have the meanings defined in the description.

The term "D₉₀" herein is an expression of particle size, which refers to the particle size corresponding to the cumulative particle size distribution of a sample reaching 90%; the term "D₅₀" is an expression of particle size, which refers to the particle size corresponding to the cumulative particle size distribution of a sample reaching 50%; and the term "D₁₀" is an expression of particle size, which refers to the particle size corresponding to the cumulative particle size distribution of a sample reaching 10%.

The term "optional" or "optionally" herein means that the event or circumstance described subsequently can, but need not, occur, and such a description includes the situation in which the event or circumstance does or does not occur.

The term "subject" refers to an animal, preferably a mammal. According to specific embodiments, the subject is a mammal including, for example, a camel, donkey, zebra, cow, pig, horse, goat, sheep, cat, dog, rat, rabbit, guinea pig, mouse, primate (for example, human). In some specific embodiments, the subject is a human. In some specific embodiments, the subject is a person who is susceptible to, suspected of suffering from, or has suffered from cancer, such as bladder cancer.

The term "treating" refers to eliminating a disease, stopping disease progression, slowing disease progression, reducing the duration of one or more symptoms associated with a disease, improving or reversing at least one measurable parameter associated with a disease, or increasing the survival rate of a subject suffering from a disease.

The term "effective amount" refers to the amount of active pharmaceutical ingredient that elicits the desired effect in a subject. In specific embodiments, those skilled in the art can determine an effective amount based on consideration of a variety of factors (for example, via clinical trials), the factors include the disease to be treated, the symptoms involved, administration route, the severity of the disease, the body weight of the patient, the immune status of the patient, and other factors known to those of skill in the art. Effective amount can be obtained from the dose-response curve derived from an animal model test system, and can be determined according to the judgment of the practitioner and each patient's circumstances. The interrelationship of dosing between animal and human is described in Freireich et al., 1966, Cancer Chemother Rep 50: 219, and the human body surface area can be approximately determined from the height and body weight of the patient. The effective amount of the active pharmaceutical ingredient of the present invention can vary from 300 mg to 1500 mg/day, and can be administered once a day or several times a day.

The progressive effects of the present invention are that:
The pharmaceutical composition of nitroxoline or a salt thereof with specific particle size of the present invention can be prepared into nitroxoline tablets with a moderate dissolution rate (the dissolution rate within 60 min is more than 75%). In addition, the resulting nitroxoline tablets have uniform content, narrow variation range in hardness, and good fluidity and compressibility.

The nitroxoline with specific particle size of the present invention is beneficial to the preparation of pharmaceutical formulation, and can further control the dissolution and absorption of the active pharmaceutical ingredient of the pharmaceutical composition in body. The obtained nitroxoline tablet is rapidly absorbed after oral administration (Tmax = 1.5 to 2.5 hours), and has high absorption rate (bioavailability is over 80%).

### DESCRPTION OF THE DRAWING

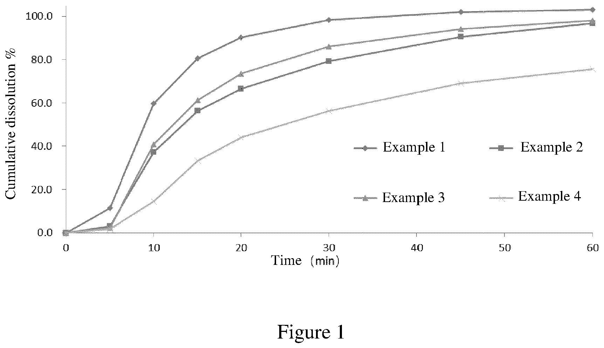

Figure 1 is the dissolution profile of the nitroxoline tablets prepared in Examples 1 to 4 of the present invention.

### DETAILED DESCRIPTION OF THE INVENTION

The present invention is further described below by the examples, but the present invention is not limited to the scope of the examples. The experimental methods without specific conditions in the following examples were carried out according to conventional methods and conditions, or according to the product description. Known reagents, solvents and materials in the examples can be synthesized using or according to methods known in the art, or are commercially available.
I. Experimental reagents:
   Nitroxoline: synthesized from the starting material 8-hydroxyquinoline through nitrosation and oxidation in two steps by reference to literatures "Chemistry of Heterocyclic Compounds (New York, NY, United States), 41(8), 1027-1030; 2005" and "International Journal of ChemTech Research, 2(1), 209-213; 2010";
   Nitroxoline lysine salt: prepared according to the method provided in the patent document (CN105228984A);
   Starch: Liaoning Dongyuan Pharmaceutical Co., Ltd. or Roquette, France;
   Pregelatinized starch: Shanghai Colorcon or Asahi Kasei, Japan;
   Microcrystalline cellulose: Dupont, USA or Mingtai Chemical Co., Ltd., Taiwan, China;
   Hydroxypropyl methylcellulose: Dow, USA or Shin-Etsu, Japan;
   Polyvinylpyrrolidone: BASF, Germany or ISP, USA;
   Lactose: Foremost Farms or Kerry, USA;
   Hydroxypropyl cellulose: Huzhou Zhanwang Pharmaceutical Co., Ltd. or Shin-Etsu, Japan;
   Sodium stearyl fumarate: Jiangxi Alpha Hi-tech Pharmaceutical CO., Ltd. or Rettenmaier, Germany;
   Low-substituted hydroxypropyl cellulose: Huzhou Zhanwang Pharmaceutical Co., Ltd. or Shin-Etsu, Japan;
   Sodium stearyl fumarate: Jiangxi Alpha Hi-tech Pharmaceutical CO., Ltd. or Rettenmaier, Germany;
   Sodium dodecyl sulfate: Hunan Jiudian Pharmaceutical Co., Ltd. or BASF, Germany;
   Silica: Huzhou Zhanwang Pharmaceutical Co., Ltd. or Evonik, Germany;
   Magnesium stearate: Huzhou Zhanwang Pharmaceutical Co., Ltd. or Peter Greven, Germany;
   Crospovidone: Chongqing Star-Tech & JRS Specialty Products Co., Ltd. or ISP, USA.
II. Experimental instruments:
   Mechanical mill: SF-130, Taizhou Tiantai Pharmacy Machinery Factory;
   Jet mill: Mini-AJM, Shenzhen Xinyite Technology Co., Ltd.;
   Malvern laser particle size analyzer: MS2000, Malvern;
   Tableting machine: Shanghai Tianfan Machinery Factory, TDP-6;
   Coating machine: BY-300B water chestnut type, Taizhou Jintai Pharmaceutical Machinery Factory;
   High shear wet granulator: G10, Shenzhen Xinyite Technology Co., Ltd.;
   Fluidized bed: WBF-3G, Chongqing Enger Granulating & Coating Technology Co., Ltd.;
   Oven: DHG-9240A, Shanghai Bluepard Instruments Co., Ltd.
III. Processing method of raw materials
   The initial particle size D₉₀ of the raw material nitroxoline or nitroxoline lysine salt was more than 100 µm. The raw material was pulverized with the mechanical mill to obtain the raw material having the particle size I, II, III or V in the following Table 1. The raw material was pulverized with the jet mill to obtain the raw material having the particle size IV in the following Table 1.
IV. Determination method for particle size

Determination was carried out on the Malvern laser particle size analyzer MS2000 by wet method according to the operation requirements of the instrument, the dispersant was an aqueous Tween 80 solution, and the mass fraction of Tween 80 in the aqueous solution was 0.5%. The results are shown in Table 1 below.

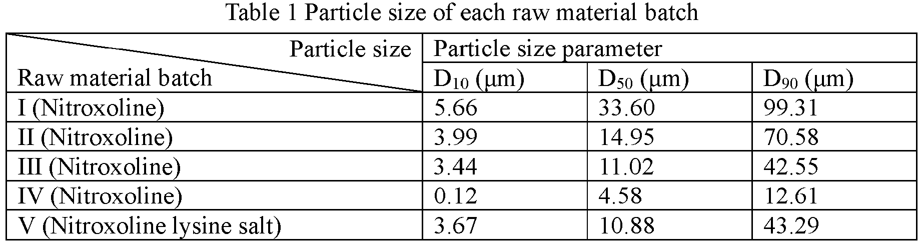

### Example 1 Preparation example I of nitroxoline tablets

The materials were weighed according to the following proportions:

| | |
|---|---|
| Nitroxoline raw material (batch I) | 100 parts by weight |
| Starch | 32 parts by weight |
| Lactose | 30 parts by weight |
| Polyvinylpyrrolidone (for pulping) | 8 parts by weight |
| Low substituted hydroxypropyl cellulose | 5 parts by weight |
| Sodium stearyl fumarate | 2.5 parts by weight |

Specifically, nitroxoline raw material (batch I, 200 g), starch (64 g), lactose (60 g), polyvinylpyrrolidone (16 g, formulated into a solution by adding 64 g of water) and low substituted hydroxypropyl cellulose (10 g) were weighed respectively, and added into the high shear wet granulator for granulation. The resulting soft material was subjected to wet milling, and dried with the fluidized bed (the moisture was controlled to 2-5%). The dried granules were subjected to dry milling, followed by the addition of sodium stearyl fumarate (5 g), and mixed for 10 min to obtain the total mixed granules. The total mixed granules were tableted by the tableting machine (φ 6.5 mm). The resulting tablets were coated by the coating machine with hydroxypropyl methylcellulose as a film coating powder (64 g, formulated into a solution by adding 336 g of water), and the weight gain was 13%. The coated nitroxoline tablets were thus prepared.

### Example 2 Preparation example II of nitroxoline tablets

The materials were weighed according to the following proportions:

| | |
|---|---|
| Nitroxoline raw material (batch II) | 100 parts by weight |
| Starch | 30 parts by weight |
| Lactose | 25 parts by weight |
| Starch (forpulping) | 2.5 parts by weight |
| Low substituted hydroxypropyl cellulose | 5 parts by weight |
| Sodium dodecyl sulfate | 4 parts by weight |

Specifically, nitroxoline raw material (batch II, 200 g), starch (60 g), lactose (50 g), starch (5 g, formulated into 5% starch pulp by adding 95 g of water) and low substituted hydroxypropyl cellulose (10 g) were weighed respectively, and added into the high shear wet granulator for granulation. The resulting soft material was subjected to wet milling, and dried with the oven (the moisture was controlled to 2-5%). The dried granules were subjected to dry milling, followed by the addition of s odium dodecyl sulfate (8 g), and mixed for 10 min to obtain the total mixed granules. The total mixed granules were tableted by the tableting machine (φ 6.5 mm). The resulting tablets were coated by the coating machine with hydroxypropyl methylcellulose as a film coating powder (64 g, formulated into a solution by adding 336 g of water), and the weight gain was 11%. The coated nitroxoline tablets were thus prepared.

### Example 3 Preparation example III of nitroxoline tablets

The materials were weighed according to the following proportions:

| | |
|---|---|
| Nitroxoline raw material (batch III) | 100 parts by weight |
| Starch | 30 parts by weight |
| Lactose | 25 parts by weight |
| Starch (for pulping) | 2 parts by weight |
| Low substituted hydroxypropyl cellulose | 5 parts by weight |
| Sodium dodecyl sulfate | 4 parts by weight |

Specifically, nitroxoline raw material (batch III, 200 g), starch (60 g), lactose (50 g), starch (4 g, formulated into 5% starch pulp by adding 76 g of water) and low substituted hydroxypropyl cellulose (10 g) were weighed respectively, and added into the high shear wet granulator for granulation. The resulting soft material was subjected to wet milling, and dried with the fluidized bed or oven (the moisture was controlled to 2-5%). The dried granules were subjected to dry milling, followed by the addition of s odium dodecyl sulfate (8 g), and mixed for 10 min to obtain the total mixed granules. The total mixed granules were tableted by the tableting machine (φ 6.5 mm). The resulting tablets were coated by the coating machine with hydroxypropyl methylcellulose as a film coating powder (64 g, formulated into a solution by adding 336 g of water), and the weight gain was 9%. The coated nitroxoline tablets were thus prepared.

### Example 4 Preparation example IV of nitroxoline tablets

The materials were weighed according to the following proportions:

| | |
|---|---|
| Nitroxoline raw material (batch IV) | 100 parts by weight |
| Microcrystalline cellulose | 30 parts by weight |
| Lactose | 25 parts by weight |
| Starch (for pulping) | 1.5 parts by weight |
| Low substituted hydroxypropyl cellulose | 5 parts by weight |
| Sodium dodecyl sulfate | 4 parts by weight |

Specifically, nitroxoline raw material (batch IV, 200 g), microcrystalline cellulose (60 g), lactose (50 g), starch (3 g, formulated into 5% starch pulp) and low substituted hydroxypropyl cellulose (10 g) were weighed respectively, and added into the high shear wet granulator for granulation. The resulting soft material was subjected to wet milling, and dried with the fluidized bed (the moisture was controlled to 2-5%). The dried granules were subjected to dry milling, followed by the addition of s odium dodecyl sulfate (8 g) was, and mixed for 10 min to obtain the total mixed granules. The total mixed granules were tableted by the tableting machine (φ 6.5 mm). The resulting tablets were coated by the coating machine with hydroxypropyl methylcellulose as a film coating powder (64 g, formulated into a solution by adding 336 g of water), and the weight gain was 10%. The coated nitroxoline tablets were thus prepared.

### Example 5 Preparation example V of nitroxoline tablets

The materials were weighed according to the following proportions:

| | |
|---|---|
| Nitroxoline lysine salt raw material (batch V) | 100 parts by weight |
| Microcrystalline cellulose | 140 parts by weight |
| Lactose | 120 parts by weight |
| Hydroxypropyl methylcellulose (for pulping) | 5 parts by weight |
| Low substituted hydroxypropyl cellulose | 5 parts by weight |
| Silica | 5 parts by weight |
| Magnesium stearate | 4 parts by weight |

Specifically, nitroxoline lysine salt (batch V, 200 g), microcrystalline cellulose (280 g), lactose (240 g), hydroxypropyl methylcellulose (10 g, formulated into a solution by adding 115 g of water) and low substituted hydroxypropyl cellulose (10 g) were weighed respectively, and added into the high shear wet granulator for granulation. The resulting soft material was subjected to wet milling, and dried with the fluidized bed (the moisture was controlled to 2-5%). The dried granules were subjected to dry milling, followed by the addition of s ilica (10 g) and magnesium stearate (8 g), and mixed for 10 min to obtain the total mixed granules. The total mixed granules were tableted by the tableting machine (φ 10 mm). The resulting tablets were coated by the coating machine with hydroxypropyl methylcellulose as a film coating powder (96 g, formulated into a solution by adding 504 g of water), and the weight gain was 8%. The coated nitroxoline tablets were thus prepared.

### Example 6 Preparation example VI of nitroxoline tablets

The materials were weighed according to the following proportions:

| | |
|---|---|
| Nitroxoline raw material (batch I) | 100 parts by weight |
| Starch | 150 parts by weight |
| Lactose | 130 parts by weight |
| Sodium dodecyl sulfate | 10 parts by weight |

Specifically, nitroxoline (batch I, 100 g), starch (150 g) and lactose (130 g) were weighed respectively, and added together with an appropriate amount of water into the high shear wet granulator for granulation. The resulting soft material was subjected to wet milling, and dried with the fluidized bed (the moisture was controlled to 2-5%). The dried granules were subjected to dry milling, followed by the addition of s odium dodecyl sulfate (10 g), and mixed for 10 min to obtain the total mixed granules. The total mixed granules were tableted by the tableting machine (φ 6.5 mm). The resulting tablets were coated by the coating machine with hydroxypropyl methylcellulose as a film coating powder (64 g, formulated into a solution by adding 336 g of water), and the weight gain was 11%. The coated nitroxoline tablets were thus prepared.

### Example 7 Preparation example VII of nitroxoline tablets

The materials were weighed according to the following proportions:

| | |
|---|---|
| Nitroxoline raw material (batch I) | 100 parts by weight |
| Starch | 30 parts by weight |
| Lactose | 20 parts by weight |
| Starch (for pulping) | 2 parts by weight |
| Low substituted hydroxypropyl cellulose | 6.7 parts by weight |
| Sodium dodecyl sulfate | 6.7 parts by weight |

Specifically, nitroxoline (batch I, 150 g), starch (45 g), lactose (30 g), starch (3 g, formulated into 5% starch pulp by adding 57 g of water) and low substituted hydroxypropyl cellulose (10 g) were weighed respectively, and added into the high shear wet granulator for granulation. The resulting soft material e was subjected to wet milling, and dried with the fluidized bed (the moisture was controlled to 2-5%). The dried granules were subjected to dry milling, followed by the addition of s odium dodecyl sulfate (10 g), and mixed for 10 min to obtain the total mixed granules. The total mixed granules were tableted by the tableting machine (φ 6.5 mm). The resulting tablets were coated by the coating machine with hydroxypropyl methylcellulose as a film coating powder (32 g, formulated into a solution by adding 168 g of water), and the weight gain was 3%. The coated nitroxoline tablets were thus prepared.

### Example 8 Preparation example VIII of nitroxoline tablets

The materials were weighed according to the following proportions:

| | |
|---|---|
| Nitroxoline raw material (batch I) | 100 parts by weight |
| Starch | 50 parts by weight |
| Lactose | 50 parts by weight |
| Starch (for pulping) | 2 parts by weight |
| Low substituted hydroxypropyl cellulose | 25 parts by weight |
| Sodium dodecyl sulfate | 5 parts by weight |

Specifically, nitroxoline (batch I, 120 g), starch (60 g), lactose (60 g), starch (2.4 g, formulated into 5% starch pulp by adding 45.6 g of water) and low substituted hydroxypropyl cellulose (30 g) were weighed respectively, and added into the high shear wet granulator for granulation. The resulting soft material was subjected to wet milling, and dried with the fluidized bed (the moisture was controlled to 2-5%). The dried granules were subjected to dry milling, followed by the addition of s odium dodecyl sulfate (6 g), and mixed for 10 min to obtain the total mixed granules. The total mixed granules were tableted by the tableting machine (φ 6.5 mm). The resulting tablets were coated by the coating machine with hydroxypropyl methylcellulose as a film coating powder (40 g, formulated into a solution by adding 210 g of water), and the weight gain was 5%. The coated nitroxoline tablets were thus prepared.

### Example 9 Preparation example IX of nitroxoline tablets

The materials were weighed according to the following proportions:

| | |
|---|---|
| Nitroxoline raw material (batch I) | 100 parts by weight |
| Starch | 50 parts by weight |
| Lactose | 50 parts by weight |
| Starch (for pulping) | 1.9 parts by weight |
| Crospovidone | 12 parts by weight |
| Sodium dodecyl sulfate | 4 parts by weight |

Specifically, nitroxoline (batch I, 130 g), starch (65 g), lactose (65 g), starch (2.5 g, formulated into 5% starch pulp by adding 47.5 g of water) and crospovidone (15.6 g) were weighed respectively, and added into the high shear wet granulator for granulation. The resulting soft material was subjected to wet milling, and dried with the fluidized bed (the moisture was controlled to 2-5%). The dried granules were subjected to dry milling, followed by the addition of s odium dodecyl sulfate (5.2 g), and mixed for 10 min to obtain the total mixed granules. The total mixed granules were tableted by the tableting machine (φ 6.58.5 mm). The resulting tablets were coated by the coating machine with hydroxypropyl methylcellulose as a film coating powder (60 g, formulated into a solution by adding 315 g of water), and the weight gain was 15%. The coated nitroxoline tablets were thus prepared.

### Experimental Example 1 Content and weight difference assay of the nitroxoline tablets of the present invention

The active pharmaceutical ingredient (nitroxoline) content in the nitroxoline tablets was determined according to high performance liquid chromatography (general rule 0512 of volume IV of the Chinese Pharmacopoeia 2015 Edition).

Content % refers to the ratio of the average content to the theoretical amount of the active pharmaceutical ingredient in each tablet.

The tablet weight difference % was calculated as follows: 20 tablets were randomly selected and weighed, and the average value was calculated; each tablet was weighed separately, and the percentage ratio of the difference between the weight of each tablet and the average value to the average value was calculated.

The content and weight difference of the nitroxoline tablets of the present invention are shown in Table 2 below.

**Table 2 Content and weight difference of the nitroxoline tablets of the present invention**

| Sample information | Content/% | Tablet weight difference/% |
|---|---|---|
| Example 1 | 98.8 | -2.09~+1.42 |
| Example 2 | 101.9 | -1.03~+1.08 |
| Example 3 | 100.3 | -1.24~+1.67 |
| Example 4 | 98.4 | -7.28~+2.26 |

Conclusion: All results meet the requirements of Chinese Pharmacopoeia.

### Experimental Example 2 Dissolution rate test of the nitroxoline tablets of the present invention

The nitroxoline tablets prepared in Example 1 to 4 were used. The operation was conducted in accordance with the test method of dissolution rate and release rate (general rule 0931 of volume IV of the Chinese Pharmacopoeia 2015 Edition), 1000 mL of 0.1 mol/L hydrochloric acid solution was used as the dissolution medium, the rotation speed was 60 revolutions per minute. At 60 minutes, the solution was taken and filtered through a membrane filter. 2 mL of the resulting filtrate was added into a 10 mL volumetric flask, and diluted with 0.1mol/L hydrochloric acid solution until the liquid level reached the mark. The resulting solution was shaked well, and used as the test solution. An appropriate amount of nitroxoline reference substance was accurately weighed, dissolved in 0.1 mol/L hydrochloric acid solution, and quantitatively diluted to prepare a solution containing about 10 µg of nitroxoline per milliliter, as the reference substance solution. According to UV-visible spectrophotometry (general rule 0401 of volume IV of the Chinese Pharmacopoeia 2015 Edition) (UV-2700, Shimadzu), the absorbance was measured at a wavelength of 369 nm, and the dissolution rate of each tablet was calculated.

The dissolution profile of the nitroxoline tablets prepared in Examples 1 to 4 is shown in Figure 1. The dissolution rates at 60 min were 103%, 97%, 98% and 76%, respectively. The above dissolution rates are moderate, and the specific explanations are as follows: The relatively rapid dissolution of an immediate-release formulation generally means that the dissolution rate at 15 min, 30 min or 45 min is more than 75%. The relatively slow dissolution of an immediate-release preparation generally means that the dissolution rate at 90 min or 120 min is more than 75%. The dissolution rate of the nitroxoline tablets obtained in the present invention at 60 min is more than 75%, that is, the nitroxoline tablets prepared from the phamaceutical composition of the present invention is an immediate-release preparation having a moderate dissolution rate.

Nitroxoline belongs to BCS class II drugs, which have low solubility and high permeability. Generally, in formulations containing this type of drugs, the smaller the particle size of the drug, the faster the dissolution of the formulation. However, the inventors unexpectedly found that the smaller the particle size of nitroxoline, the slower the dissolution of the formulation. As can be seen from Figure 1, along with the reduction of the particle size of nitroxoline, the overall dissolution of the nitroxoline tablets of the present invention is slowed down. This is inconsistent with the dissolution profiles exhibited by conventional formulations containing such drugs. As for Examples 2 and 3, since the particle sizes of the two are relatively close, the two show relatively similar dissolution.

### Experimental Example 3 Hardness test of the nitroxoline tablets of the present invention

The drug was placed into a hardness tester (SY-3, Yellow Sea) according to the operation requirements, and subjected to hardness test. 10 tablets were measured each time, that is, serial numbers 1 to 10. The hardness of the nitroxoline tablets prepared in Examples 1 to 4 is shown in Table 3 below.

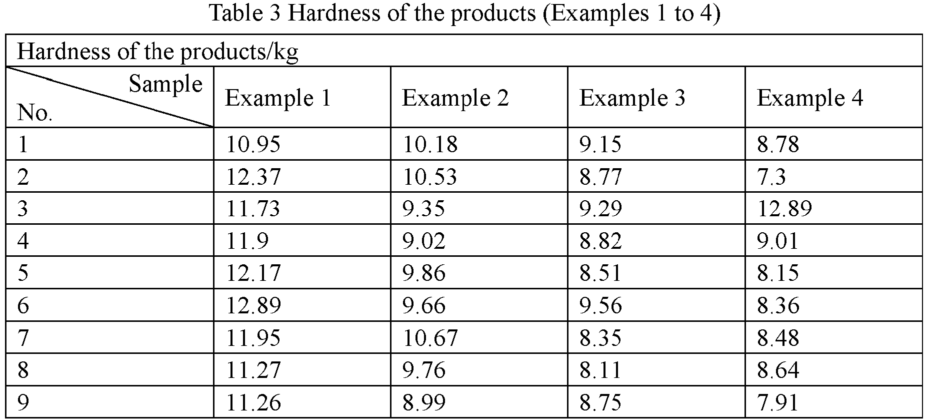

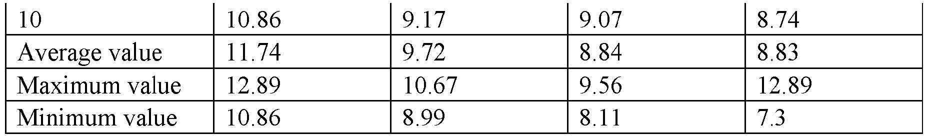

It can be seen from the results of hardness test in the table above that the hardness of the products of Examples 1 to 4 is within the expected range, and the variation range of hardness is narrow.

### Experimental Example 4 Fluidity test and compressibility test

In this experiment, the fluidity of the particles was evaluated by measuring the Carr Index of the mixed particles, and the compressibility of the particles was evaluated by the Hsusner Ratio.

Carr Index refers to the percentage difference between the tap density and the bulk density of the particles. ≤10% indicates fairly excellent fluidity, 11 to 15% indicates excellent fluidity, 16 to 20% indicates good fluidity, 21 to 25% indicates passable fluidity, 26 to 31% indicates poor fluidity, and above 32% indicates very poor fluidity.

Hsusner Ratio refers to the ratio of the tap density to the bulk density of the particles. 1.00 to 1.11 indicates fairly excellent compressibility, 1.12 to 1.18 indicates excellent compressibility, 1.19 to 1.25 indicates good compressibility, 1.26 to 1.34 indicates passable fluidity, 1.35 to 1.45 indicates poor fluidity, and 1.46 to 1.59 indicates very poor fluidity.

Test method of the bulk density: About 20 g of the granules were weighed, and slowly added into a 100 mL measuring cylinder. The measuring cylinder was initially inclined, and slowly placed upright after the addition, vibration should be avoided during the process. The volume was read, and the bulk density was obtained by dividing the particle weight by the bulk volume.

Test method of the tap density: After the bulk density test, the measuring cylinder was placed on a tapping instrument, and tapped for 500 times. The volume value was read, and the tap density was obtained by dividing the particle weight by the tap volume.

Before tableting, the total mixed granules of Examples 1 to 4 were taken and tested as described above. The resulting Carr Index and Hsusner Ratio are shown in Table 4 below.

**Table 4 Carr Index and Hsusner Ratio of the nitroxoline total mixed granules of Examples 1 to 4**

| Sample | Particle mass/(g) | Bulk volume/(cm³) | Bulk density/(g/m³) | Tap volume/(cm³) | Tap density/(g/m³) | Carr Index/% | Hsusner Ratio |
|---|---|---|---|---|---|---|---|
| Example 1 | 20.6101 | 43.0 | 0.4793 | 38.0 | 0.5424 | 13.2 | 1.13 |
| Example 2 | 20.8697 | 40.0 | 0.5217 | 33.0 | 0.6324 | 21.2 | 1.21 |
| Example 3 | 22.5043 | 44.0 | 0.5115 | 38.0 | 0.5922 | 15.7 | 1.16 |
| Example 4 | 20.6105 | 36.0 | 0.5725 | 29.0 | 0.7107 | 24.1 | 1.24 |

It can be seen from Table 4 above that the Carr Indexes of the nitroxoline total mixed particles of the present invention are all less than 25%, indicating that the particles have good fluidity. Moreover, the Hsusner Ratios of the nitroxoline total mixed particles of the present invention are less than 1.25, indicating that the compressibility of the particles is good or better.

In summary, the pharmaceutical composition of nitroxoline or a salt thereof with specific particle size provided in the present invention allows the resulting nitroxoline tablets to have a moderate dissolution rate (the dissolution rate within 60 min is more than 75%), uniform content, narrow variation range in hardness, and good fluidity and compressibility. Moreover, the nitroxoline tablets are not only conducive to the absorption of active pharmaceutical ingredients in the body, but also have a simple preparation process that is suitable for industrial production.

## Claims

1. A pharmaceutical composition, wherein the pharmaceutical composition comprises an active pharmaceutical ingredient and a pharmaceutically acceptable carrier, the active pharmaceutical ingredient is nitroxoline or a pharmaceutically acceptable salt thereof, **characterized in that** the particle size D₉₀ of the active pharmaceutical ingredient is 10 to 100 µm, determination of particle size is carried out on the Malvern laser particle size analyzer MS2000 by wet method according to the operation requirements of the instrument, the dispersant was an aqueous Tween 80 solution, and the mass fraction of Tween 80 in the aqueous solution was 0.5%;
wherein, the active pharmaceutical ingredient is present in an amount of 20 to 65 parts by weight or 20 to 60 parts by weight, preferably 25 to 60 parts by weight, more preferably 40 to 61 parts by weight, and further more preferably 56 to 61 parts by weight per 100 parts by weight of the pharmaceutical composition;
the carrier comprises a filler; the filler is one or more of starch, pregelatinized starch, partially pregelatinized starch, lactose, sucrose, mannitol, sorbitol, hydrous calcium phosphate, anhydrous calcium phosphate and microcrystalline cellulose, and more preferably one or more of starch, lactose and microcrystalline cellulose; the filler is preferably present in an amount of 30 to 300 parts by weight, more preferably 50 to 100 parts by weight, further more preferably 30 to 90 parts by weight, and still further more preferably 30 to 75 parts by weight per 100 parts by weight of the active pharmaceutical ingredient;
the carrier comprises a binder; the binder is one or more of hydroxypropyl cellulose, hydroxypropyl methylcellulose, polyvinylpyrrolidone and starch, more preferably one or more of polyvinylpyrrolidone, hydroxypropyl methylcellulose and starch, and further more preferably polyvinylpyrrolidone and/or starch; the binder is preferably present in an amount of 0 to 100 parts by weight, and not 0, more preferably 2 to 50 parts by weight, and further more preferably 30 to 50 parts by weight per 100 parts by weight of the active pharmaceutical ingredient;
the carrier comprises a disintegrant; the disintegrant is one or more of low-substituted hydroxypropyl cellulose, calcium carboxymethyl cellulose, sodium carboxymethyl starch, croscarmellose sodium and crospovidone, more preferably low-substituted hydroxypropyl cellulose and/or crospovidone, and further more preferably low-substituted hydroxypropyl cellulose; the disintegrant is preferably present in an amount of 0 to 100 parts by weight, and not 0, more preferably 1 to 25 parts by weight or 5 to 25 parts by weight, further more preferably 1 to 10 parts by weight, 2 to 10 parts by weight or 5 to 10 parts by weight, and still further more preferably 5 to 6 parts by weight per 100 parts by weight of the active pharmaceutical ingredient;
the carrier comprises a lubricant; the lubricant is one or more of magnesium stearate, sodium stearyl fumarate and sodium dodecyl sulfate, and more preferably sodium stearyl fumarate and/or sodium dodecyl sulfate; the lubricant is preferably present in an amount of 0.25 to 20 parts by weight, more preferably 0.5 to 5 parts by weight, and further more preferably 2.5 to 4 parts by weight per 100 parts by weight of the active pharmaceutical ingredient.

2. The pharmaceutical composition according to claim 1, **characterized in that** the particle size D₉₀ of the active pharmaceutical ingredient is 10 to 70 µm, and preferably 40 to 70 µm;
and/or, the particle size D₅₀ of the active pharmaceutical ingredient is 4 to 50 µm, preferably 4 to 40 µm or 4 to 35 µm, and more preferably 10 to 40 µm;
and/or, the particle size D₁₀ of the active pharmaceutical ingredient is 0 to 10 µm, and is not 0, preferably 0.1 to 6 µm or 2 to 10 µm, and more preferably 2 to 6 µm.

3. The pharmaceutical composition according to claim 1 or 2, **characterized in that** the carrier comprises a glidant; the glidant is preferably one or more of silica, talc and sodium dodecyl sulfate, more preferably silica and/or sodium dodecyl sulfate, and further more preferably sodium dodecyl sulfate; the glidant is preferably present in an amount of 0 to 20 parts by weight, and not 0, more preferably 0.5 to 10 parts by weight, and further more preferably 4 to 10 parts by weight per 100 parts by weight of the active pharmaceutical ingredient.

4. The pharmaceutical composition according to claim 1 or 2, **characterized in that** the carrier comprises a filler and a binder, the types of the filler and the binder are as defined in claim 1, and the filler and the binder are present in a total amount of 30 to 300 parts by weight, preferably 32 to 280 parts by weight, more preferably 32 to 140 parts by weight, and further more preferably 56.5 to 70 parts by weight per 100 parts by weight of the active pharmaceutical ingredient;
and/or, the carrier comprises a lubricant and a glidant, the types of the lubricant and the glidant are as defined in claim 1, and the lubricant and the glidant are present in a total amount of 1 to 10 parts by weight, preferably 1 to 15 parts by weight, more preferably 2.5 to 10 parts by weight, and further more preferably 2.5 to 4 parts by weight per 100 parts by weight of the active pharmaceutical ingredient.

5. The pharmaceutical composition according to any one of claims 1 to 4, **characterized in that** the pharmaceutical composition also comprises a coating agent; the coating agent is preferably hydroxypropyl methylcellulose and/or polyvinyl alcohol, and more preferably hydroxypropyl methylcellulose; the coating agent is preferably present in the pharmaceutical composition in an amount of 3% to 15% by mass, and more preferably 9% to 13% by mass relative to the mass of the uncoated pharmaceutical composition.

6. The pharmaceutical composition according to claim 1, **characterized in that** the particle size D₅₀ of the active pharmaceutical ingredient is 4 to 35 µm, the particle size D₁₀ of the active pharmaceutical ingredient is 0.1 to 6 µm; the active pharmaceutical ingredient is present in an amount of 56 to 61 parts by weight per 100 parts by weight of the pharmaceutical composition;
the carrier comprises a filler and a binder, the filler is one or more of starch, lactose and microcrystalline cellulose, the binder is polyvinylpyrrolidone and/or starch; the filler and the binder are present in a total amount of 56.5 to 70 parts by weight per 100 parts by weight of the active pharmaceutical ingredient;
the carrier comprises a disintegrant, the disintegrant is low-substituted hydroxypropyl cellulose; the disintegrant is present in an amount of 5 to 6 parts by weight per 100 parts by weight of the active pharmaceutical ingredient;
the carrier comprises adjuvant I, the adjuvant I is sodium stearyl fumarate and/or sodium dodecyl sulfate; the adjuvant I is present in an amount of 2.5 to 4 parts by weight per 100 parts by weight of the active pharmaceutical ingredient;
the pharmaceutical composition also comprises a coating agent, the coating agent is hydroxypropyl methylcellulose, the coating agent is present in the pharmaceutical composition in an amount of 9% to 13% by mass relative to the mass of the uncoated pharmaceutical composition.

7. A nitroxoline tablet, **characterized in that** the nitroxoline tablet is prepared from the pharmaceutical composition according to any one of claims 1 to 6.

8. A method for preparing a nitroxoline tablet, **characterized in that** the pharmaceutical composition in the method is the pharmaceutical composition according to any one of claims 1 to 6, the pharmaceutical composition comprises adjuvant II, the adjuvant II is a lubricant and/or a glidant;
the method comprises the following steps of: subjecting the components in the pharmaceutical composition except adjuvant II to wet granulation, wet milling, drying and dry milling, adding adjuvant II, subjecting the resulting mixture to total mixing, and tableting to obtain the nitroxoline tablet;
when the pharmaceutical composition comprises a coating agent, coating is performed after tableting.

9. The pharmaceutical composition according to any one of claims 1 to 6 and/or the nitroxoline tablet according to claim 7 for use in the treatment of cancer; preferably bladder cancer.

10. A nitroxoline solid tablet composition, **characterized in that** it comprises an active pharmaceutical ingredient uniformly dispersed therein and a pharmaceutically acceptable carrier, the active pharmaceutical ingredient is nitroxoline or a pharmaceutically acceptable salt thereof, the particle size D₉₀ of the active pharmaceutical ingredient is 10 to 100 µm, preferably 10 to 70 µm, and more preferably 40 to 70 µm, determination of particle size is carried out on the Malvern laser particle size analyzer MS2000 by wet method according to the operation requirements of the instrument, the dispersant was an aqueous Tween 80 solution, and the mass fraction of Tween 80 in the aqueous solution was 0.5%;
the active pharmaceutical ingredient is present in an amount of 20 to 60 parts by weight, and preferably 25 to 60 parts by weight per 100 parts by weight of the pharmaceutical composition;
wherein, the pharmaceutically acceptable carrier comprises filler, disintegrant, binder, glidant and lubricant;
wherein, the filler is one or more of starch, pregelatinized starch, partially pregelatinized starch, lactose, sucrose, mannitol, sorbitol, hydrous calcium phosphate, anhydrous calcium phosphate and microcrystalline cellulose; preferably, the filler is present in an amount of 30 to 300 parts by weight, preferably 50 to 100 parts by weight, more preferably 30 to 90 parts by weight, and further more preferably 30 to 75 parts by weight per 100 parts by weight of the active pharmaceutical ingredient;
the disintegrant is one or more of low-substituted hydroxypropyl cellulose, calcium carboxymethyl cellulose, sodium carboxymethyl starch, croscarmellose sodium and crospovidone; preferably, the disintegrant is present in an amount of 0 to 100 parts by weight, and not 0, preferably 5 to 25 parts by weight, and more preferably 5 to 10 parts by weight per 100 parts by weight of the active pharmaceutical ingredient;
the binder is one or more of hydroxypropyl cellulose, hydroxypropyl methylcellulose, polyvinylpyrrolidone and starch; preferably, the binder is present in an amount of 0 to 100 parts by weight, and not 0, preferably 2 to 50 parts by weight, and more preferably 30 to 50 parts by weight per 100 parts by weight of the active pharmaceutical ingredient;
the lubricant is one or more of magnesium stearate, sodium stearyl fumarate and sodium dodecyl sulfate; preferably, the lubricant is present in an amount of 0.25 to 20 parts by weight, preferably 1 to 5 parts by weight, and more preferably 2.5 to 4 parts by weight per 100 parts by weight of the active pharmaceutical ingredient.

11. The nitroxoline solid tablet composition according to claim 10, **characterized in that** the particle size D₅₀ of the active pharmaceutical ingredient is 4 to 50 µm, preferably 4 to 40 µm, and more preferably 10 to 40 µm;
and/or, the particle size D₁₀ of the active pharmaceutical ingredient is 0 to 10 µm, and is not 0, preferably 2 to 10 µm, and more preferably 2 to 6 µm.

12. The nitroxoline solid tablet composition according to claim 10 or 11, **characterized in that** the pharmaceutically acceptable carrier further comprises one or more of glidant, colorant, pH adjuster, surfactant, stabilizer and fragrance.

13. The nitroxoline solid tablet composition according to claim 10 or 11, **characterized in that**
the pharmaceutically acceptable carrier further comprises the glidant, wherein the glidant is one or more of silica, talc and sodium dodecyl sulfate; preferably, the glidant is present in an amount of 0 to 20 parts by weight, and not 0, preferably 0.5 to 10 parts by weight, and more preferably 4 to 10 parts by weight per 100 parts by weight of the active pharmaceutical ingredient.

14. The nitroxoline solid tablet composition according to any one of claims 10 to 13, **characterized in that** the nitroxoline solid tablet composition further comprising a film coating material;
the film coating material is preferably hydroxypropyl methylcellulose and/or polyvinyl alcohol;
the weight gain of the film coating material is preferably 3% to 15% per 100 parts by weight of the active pharmaceutical ingredient.

15. The nitroxoline tablet of claim 7, **characterized in that** it comprises:
| | |
|---|---|
| nitroxoline | 100 parts by weight, |
| filler | 30 to 300 parts by weight, preferably 50 to 100 parts by weight, |
| disintegrant | 0 to 100 parts by weight, preferably 5 to 25 or 5 to 10 parts by weight, |
| binder | 0 to 100 parts by weight, preferably 5 to 25 or 2 to 50 parts by weight, |
| lubricant | 0.25 to 20 parts by weight, preferably 2.5 to 4 or 0.5 to 5 parts by weight, |
| glidant | 0 to 20 parts by weight, preferably 4 to 10 or 0.5 to 10 parts by weight, and |
| optional film coating powder | 3% to 15% weight gain per 100 parts by weight of nitroxoline, and |
the particle size D₉₀ of nitroxoline is 10 to 100 µm, preferably 10 to 70 µm, and more preferably 40 to 70 µm, determination of particle size is carried out on the Malvern laser particle size analyzer MS2000 by wet method according to the operation requirements of the instrument, the dispersant was an aqueous Tween 80 solution, and the mass fraction of Tween 80 in the aqueous solution was 0.5%;
the types of the filler, disintegrant, binder, lubricant and glidant are as defined in claim 10.

## Patentansprüche

1. Pharmazeutische Zusammensetzung, wobei die pharmazeutische Zusammensetzung einen pharmazeutischen Wirkstoff und einen pharmazeutisch unbedenklichen Träger umfasst, der pharmazeutische Wirkstoff Nitroxolin oder ein pharmazeutisch unbedenkliches Salz davon ist, **dadurch gekennzeichnet, dass** die Partikelgröße D₉₀ des pharmazeutischen Wirkstoffs 10 bis 100 µm beträgt, die Bestimmung der Partikelgröße auf dem Laserpartikelgrößenanalysator MS2000 von Malvern nach dem Nassverfahren gemäß den Betriebsanforderungen des Instruments durchgeführt wird, das Dispergiermittel eine wässrige Tween-80-Lösung war und der Massenanteil von Tween 80 in der wässrigen Lösung 0,5 % betrug;
wobei der pharmazeutische Wirkstoff in einer Menge von 20 bis 65 Gewichtsteilen oder 20 bis 60 Gewichtsteilen, vorzugsweise 25 bis 60 Gewichtsteilen, weiter bevorzugt 40 bis 61 Gewichtsteilen und noch weiter bevorzugt 56 bis 61 Gewichtsteilen pro 100 Gewichtsteile der pharmazeutischen Zusammensetzung vorliegt;
der Träger einen Füllstoff umfasst; der Füllstoff eines oder mehrere von Stärke, vorverkleisterter Stärke, teilweise vorverkleisteter Stärke, Lactose, Saccharose, Mannitol, Sorbitol, hydratisiertem Calciumphosphat, wasserfreiem Calciumphosphat und mikrokristalliner Cellulose und besonders bevorzugt Stärke, Lactose und mikrokristalline Cellulose ist; der Füllstoff vorzugsweise in einer Menge von 30 bis 300 Gewichtsteilen, weiter bevorzugt 50 bis 100 Gewichtsteilen, noch weiter bevorzugt 30 bis 90 Gewichtsteilen und noch weiter bevorzugt 30 bis 75 Gewichtsteilen pro 100 Gewichtsteile des pharmazeutischen Wirkstoffs vorliegt;
der Träger ein Bindemittel umfasst; das Bindemittel eines oder mehrere von Hydroxypropylcellulose, Hydroxypropylmethylcellulose, Polyvinylpyrrolidon und Stärke, weiter bevorzugt eines oder mehrere von Polyvinylpyrrolidon, Hydroxypropylmethylcellulose und Stärke und noch weiter bevorzugt Polyvinylpyrrolidon und/oder Stärke ist; das Bindemittel vorzugsweise in einer Menge von 0 bis 100 Gewichtsteilen und nicht 0, weiter bevorzugt 2 bis 50 Gewichtsteilen und noch weiter bevorzugt 30 bis 50 Gewichtsteilen pro 100 Gewichtsteile des pharmazeutischen Wirkstoffs vorliegt;
der Träger ein Sprengmittel umfasst; das Sprengmittel eines oder mehrere von niedrig substituierter Hydroxypropylcellulose, Calciumcarboxymethylcellulose, Natriumcarboxymethylstärke, Croscarmellose-Natrium und Crospovidon, weiter bevorzugt niedrig substituierte Hydroxypropylcellulose und/oder Crospovidon und noch weiter bevorzugt niedrig substituierte Hydroxypropylcellulose ist; das Sprengmittel vorzugsweise in einer Menge von 0 bis 100 Gewichtsteilen und nicht 0, weiter bevorzugt 1 bis 25 Gewichtsteilen oder 5 bis 25 Gewichtsteilen, noch weiter bevorzugt 1 bis 10 Gewichtsteilen, 2 bis 10 Gewichtsteilen oder 5 bis 10 Gewichtsteilen und noch weiter bevorzugt 5 bis 6 Gewichtsteilen pro 100 Gewichtsteilen des pharmazeutischen Wirkstoffs vorliegt;
der Träger ein Schmiermittel umfasst; das Schmiermittel eines oder mehrere von Magnesiumstearat, Natriumstearylfumarat und Natriumdodecylsulfat, besonders bevorzugt Natriumstearylfumarat und/oder Natriumdodecylsulfat, ist; das Schmiermittel vorzugsweise in einer Menge von 0,25 bis 20 Gewichtsteilen, weiter bevorzugt 0,5 bis 5 Gewichtsteilen und noch weiter bevorzugt 2,5 bis 4 Gewichtsteilen pro 100 Gewichtsteile des pharmazeutischen Wirkstoffs vorliegt.

2. Pharmazeutische Zusammensetzung nach Anspruch 1, **dadurch gekennzeichnet, dass** die Partikelgröße D₉₀ des pharmazeutischen Wirkstoffs 10 bis 70 µm und vorzugsweise 40 bis 70 µm beträgt;
und/oder die Partikelgröße D₅₀ des pharmazeutischen Wirkstoffs 4 bis 50 µm, vorzugsweise 4 bis 40 µm oder 4 bis 35 µm und weiter bevorzugt 10 bis 40 µm beträgt;
und/oder die Teilchengröße D₁₀ des pharmazeutischen Wirkstoffs 0 bis 10 µm und nicht 0, vorzugsweise 0,1 bis 6 µm oder 2 bis 10 µm und weiter bevorzugt 2 bis 6 µm beträgt.

3. Pharmazeutische Zusammensetzung nach Anspruch 1 oder 2, **dadurch gekennzeichnet, dass** der Träger ein Gleitmittel umfasst; das Gleitmittel vorzugsweise eines oder mehrere von Siliciumdioxid, Talk und Natriumdodecylsulfat, besonders bevorzugt Siliciumdioxid und/oder Natriumdodecylsulfat und weiter besonders bevorzugt Natriumdodecylsulfat ist; das Gleitmittel vorzugsweise in einer Menge von 0 bis 20 Gewichtsteilen und nicht 0, weiter bevorzugt 0,5 bis 10 Gewichtsteilen und noch weiter bevorzugt 4 bis 10 Gewichtsteilen pro 100 Gewichtsteile des pharmazeutischen Wirkstoffs vorliegt.

4. Pharmazeutische Zusammensetzung nach Anspruch 1 oder 2, **dadurch gekennzeichnet, dass** der Träger einen Füllstoff und ein Bindemittel umfasst, die Typen des Füllstoffs und des Bindemittels wie in Anspruch 1 definiert sind und der Füllstoff und das Bindemittel in einer Gesamtmenge von 30 bis 300 Gewichtsteilen, vorzugsweise 32 bis 280 Gewichtsteilen, weiter bevorzugt 32 bis 140 Gewichtsteilen und noch weiter bevorzugt 56,5 bis 70 Gewichtsteile pro 100 Gewichtsteile des pharmazeutischen Wirkstoffs vorliegen;
und/oder der Träger ein Schmiermittel und ein Gleitmittel umfasst, die Arten des Schmiermittels und des Gleitmittels wie in Anspruch 1 definiert sind und das Schmiermittel und das Gleitmittel in einer Gesamtmenge von 1 bis 10 Gewichtsteilen, vorzugsweise 1 bis 15 Gewichtsteilen, weiter bevorzugt 2,5 bis 10 Gewichtsteilen und noch weiter bevorzugt 2,5 bis 4 Gewichtsteilen pro 100 Gewichtsteile des pharmazeutischen Wirkstoffs vorliegen.

5. Pharmazeutische Zusammensetzung nach einem der Ansprüche 1 bis 4, **dadurch gekennzeichnet, dass** die pharmazeutische Zusammensetzung auch ein Beschichtungsmittel umfasst; das Beschichtungsmittel vorzugsweise Hydroxypropylmethylcellulose und/oder Polyvinylalkohol und besonders bevorzugt Hydroxypropylmethylcellulose ist; das Beschichtungsmittel vorzugsweise in der pharmazeutischen Zusammensetzung in einer Menge von 3 bis 15 Massen-% und weiter bevorzugt 9 bis 13 Massen-%, bezogen auf die Masse der unbeschichteten pharmazeutischen Zusammensetzung, vorliegt.

6. Pharmazeutische Zusammensetzung nach Anspruch 1, **dadurch gekennzeichnet, dass** die Partikelgröße D₅₀ des pharmazeutischen Wirkstoffs 4 bis 35 µm beträgt, die Partikelgröße D₁₀ des pharmazeutischen Wirkstoffs 0,1 bis 6 µm beträgt; der pharmazeutische Wirkstoff in einer Menge von 56 bis 61 Gewichtsteilen pro 100 Gewichtsteile der pharmazeutischen Zusammensetzung vorliegt;
der Träger einen Füllstoff und ein Bindemittel umfasst, der Füllstoff eines oder mehrere von Stärke, Lactose und mikrokristalliner Cellulose ist, das Bindemittel Polyvinylpyrrolidon und/oder Stärke ist; der Füllstoff und das Bindemittel in einer Gesamtmenge von 56,5 bis 70 Gewichtsteilen pro 100 Gewichtsteile des pharmazeutischen Wirkstoffs vorliegen;
der Träger ein Sprengmittel umfasst, das Sprengmittel niedrig substituierte Hydroxypropylcellulose ist; das Sprengmittel in einer Menge von 5 bis 6 Gewichtsteilen pro 100 Gewichtsteile des pharmazeutischen Wirkstoffs vorliegt;
der Träger Adjuvans I umfasst, das Adjuvans I Natriumstearylfumarat und/oder Natriumdodecylsulfat ist; das Adjuvans I in einer Menge von 2,5 bis 4 Gewichtsteilen pro 100 Gewichtsteile des pharmazeutischen Wirkstoffs vorliegt;
die pharmazeutische Zusammensetzung auch ein Beschichtungsmittel umfasst, das Beschichtungsmittel Hydroxypropylmethylcellulose ist, das Beschichtungsmittel in der pharmazeutischen Zusammensetzung in einer Menge von 9 bis 13 Massen-%, bezogen auf die Masse der unbeschichteten pharmazeutischen Zusammensetzung, vorliegt.

7. Nitroxolintablette, **dadurch gekennzeichnet, dass** die Nitroxolintablette aus der pharmazeutischen Zusammensetzung nach einem der Ansprüche 1 bis 6 hergestellt ist.

8. Verfahren zur Herstellung einer Nitroxolintablette, **dadurch gekennzeichnet, dass** die pharmazeutische Zusammensetzung bei dem Verfahren die pharmazeutische Zusammensetzung nach einem der Ansprüche 1 bis 6 ist, die pharmazeutische Zusammensetzung Adjuvans II umfasst, das Adjuvans II ein Schmiermittel und/oder ein Gleitmittel ist;
wobei man bei dem Verfahren die Komponenten in der pharmazeutischen Zusammensetzung außer Adjuvans II die folgenden Schritte unterwirft: Nassgranulierung, Nassmahlung, Trocknung und Trockenmahlung, Zugabe van Adjuvans II, Gesamtmischung und Tablettierung die erhaltene Mischung wobei man die Nitroxolintablette erhält;
dann, wenn die pharmazeutische Zusammensetzung ein Beschichtungsmittel umfasst, erfolgt nach dem Tablettieren eine Beschichtung.

9. Pharmazeutische Zusammensetzung nach einem der Ansprüche 1 bis 6 und/oder Nitroxolintablette nach Anspruch 7 zur Verwendung bei der Behandlung von Krebs; vorzugsweise Blasenkrebs.

10. Feste Nitroxolintablettenzusammensetzung, **dadurch gekennzeichnet, dass** sie einen darin einheitlich dispergierten pharmazeutischen Wirkstoff und einen pharmazeutisch unbedenklichen Träger umfasst, der pharmazeutische Wirkstoff Nitroxolin oder ein pharmazeutisch unbedenkliches Salz davon ist, die Teilchengröße D₉₀ des pharmazeutischen Wirkstoffs 10 bis 100 µm, vorzugsweise 10 bis 70 µm und weiter bevorzugt 40 bis 70 µm beträgt, die Bestimmung der Partikelgröße auf dem Laserpartikelgrößenanalysator MS2000 von Malvern nach dem Nassverfahren gemäß den Betriebsanforderungen des Instruments durchgeführt wird, das Dispergiermittel eine wässrige Tween-80-Lösung war und der Massenanteil von Tween 80 in der wässrigen Lösung 0,5 % betrug;
der pharmazeutische Wirkstoff in einer Menge von 20 bis 60 Gewichtsteilen und vorzugsweise 25 bis 60 Gewichtsteilen pro 100 Gewichtsteile der pharmazeutischen Zusammensetzung vorliegt;
wobei der pharmazeutisch unbedenkliche Träger Füllstoff, Sprengmittel, Bindemittel, Gleitmittel und Schmiermittel umfasst;
wobei der Füllstoff eines oder mehrere von Stärke, vorverkleisteter Stärke, teilweise vorverkleisteter Stärke, Lactose, Saccharose, Mannitol, Sorbitol, hydratisiertem Calciumphosphat, wasserfreiem Calciumphosphat und mikrokristalliner Cellulose ist; der Füllstoff vorzugsweise in einer Menge von 30 bis 300 Gewichtsteilen, vorzugsweise 50 bis 100 Gewichtsteilen, weiter bevorzugt 30 bis 90 Gewichtsteilen und noch weiter bevorzugt 30 bis 75 Gewichtsteilen pro 100 Gewichtsteile des pharmazeutischen Wirkstoffs vorliegt;
das Sprengmittel eines oder mehrere von niedrig substituierter Hydroxypropylcellulose, Calciumcarboxymethylcellulose, Natriumcarboxymethylstärke, Croscarmellose-Natrium und Crospovidon ist; das Sprengmittel vorzugsweise in einer Menge von 0 bis 100 Gewichtsteilen und nicht 0, vorzugsweise 5 bis 25 Gewichtsteilen und weiter bevorzugt 5 bis 10 Gewichtsteilen pro 100 Gewichtsteile des pharmazeutischen Wirkstoffs vorliegt;
das Bindemittel eines oder mehrere von Hydroxypropylcellulose, Hydroxypropylmethylcellulose, Polyvinylpyrrolidon und Stärke ist; das Bindemittel vorzugsweise in einer Menge von 0 bis 100 Gewichtsteilen und nicht 0, vorzugsweise 2 bis 50 Gewichtsteilen und weiter bevorzugt 30 bis 50 Gewichtsteilen pro 100 Gewichtsteile des pharmazeutischen Wirkstoffs vorliegt;
das Schmiermittel eines oder mehrere von Magnesiumstearat, Natriumstearylfumarat und Natriumdodecylsulfat ist; das Schmiermittel vorzugsweise in einer Menge von 0,25 bis 20 Gewichtsteilen, vorzugsweise 1 bis 5 Gewichtsteilen und weiter bevorzugt 2,5 bis 4 Gewichtsteilen pro 100 Gewichtsteile des pharmazeutischen Wirkstoffs vorliegt.

11. Feste Nitroxolin-Tablettenzusammensetzung nach Anspruch 10, **dadurch gekennzeichnet, dass** die Partikelgröße D₅₀ des pharmazeutischen Wirkstoffs 4 bis 50 µm, vorzugsweise 4 bis 40 µm und weiter bevorzugt 10 bis 40 µm beträgt;
und/oder die Teilchengröße D₁₀ des pharmazeutischen Wirkstoffs 0 bis 10 µm und nicht 0, vorzugsweise 2 bis 10 µm und weiter bevorzugt 2 bis 6 µm beträgt.

12. Feste Nitroxolin-Tablettenzusammensetzung nach Anspruch 10 oder 11, **dadurch gekennzeichnet, dass** der pharmazeutisch unbedenkliche Träger ferner eines oder mehrere von Gleitmittel, Farbmittel, Mittel zur Einstellung des pH-Werts, Tensid, Stabilisator und Duftstoff umfasst.

13. Feste Nitroxolin-Tablettenzusammensetzung nach Anspruch 10 oder 11, **dadurch gekennzeichnet, dass**
der pharmazeutisch unbedenkliche Träger ferner das Gleitmittel umfasst, wobei das Gleitmittel eines oder mehrere von Siliciumdioxid, Talk und Natriumdodecylsulfat ist; das Gleitmittel vorzugsweise in einer Menge von 0 bis 20 Gewichtsteilen und nicht 0, vorzugsweise 0,5 bis 10 Gewichtsteilen und weiter bevorzugt 4 bis 10 Gewichtsteilen pro 100 Gewichtsteile des pharmazeutischen Wirkstoffs vorliegt.

14. Feste Nitroxolin-Tablettenzusammensetzung nach einem der Ansprüche 10 bis 13, **dadurch gekennzeichnet, dass** die feste Nitroxolin-Tablettenzusammensetzung ferner ein Filmbeschichtungsmaterial umfasst;
das Filmbeschichtungsmaterial vorzugsweise Hydroxypropylmethylcellulose und/oder Polyvinylalkohol ist;
die Gewichtszunahme des Filmbeschichtungsmaterials vorzugsweise 3 bis 15 % pro 100 Gewichtsteile des pharmazeutischen Wirkstoffs beträgt.

15. Nitroxolintablette nach Anspruch 7, **dadurch gekennzeichnet, dass** sie Folgendes umfasst:
Nitroxolin 100 Gewichtsteile,
Füllstoff 30 bis 300 Gewichtsteile, vorzugsweise 50 bis 100 Gewichtsteile,
Sprengmittel 0 bis 100 Gewichtsteile, vorzugsweise 5 bis 25 oder 5 bis 10 Gewichtsteile,
Bindemittel 0 bis 100 Gewichtsteile, vorzugsweise 5 bis 25 oder 2 bis 50 Gewichtsteile,
Schmiermittel 0,25 bis 20 Gewichtsteile, vorzugsweise 2,5 bis 4 oder 0,5 bis 5 Gewichtsteile,
Gleitmittel 0 bis 20 Gewichtsteile, vorzugsweise 4 bis 10 oder 0,5 bis 10 Gewichtsteile, und
fakultatives Filmbeschichtungspulver 3 bis 15 % Gewichtszunahme pro 100 Gewichtsteile Nitroxolin, und
die Teilchengröße D₉₀ von Nitroxolin 10 bis 100 µm, vorzugsweise 10 bis 70 µm und weiter bevorzugt 40 bis 70 µm beträgt, die Bestimmung der Partikelgröße auf dem Laserpartikelgrößenanalysator MS2000 von Malvern nach dem Nassverfahren gemäß den Betriebsanforderungen des Instruments durchgeführt wird, das Dispergiermittel eine wässrige Tween-80-Lösung war und der Massenanteil von Tween 80 in der wässrigen Lösung 0,5 % betrug;
die Typen des Füllstoffs, Sprengmittels, Bindemittels, Schmiermittels und Gleitmittels wie in Anspruch 10 definiert sind.

## Revendications

1. Composition pharmaceutique, dans laquelle la composition pharmaceutique comprend un ingrédient pharmaceutique actif et un support pharmaceutiquement acceptable, l'ingrédient pharmaceutique actif est la nitroxoline ou un sel pharmaceutiquement acceptable correspondant, **caractérisée en ce que** la taille de particule D₉₀ de l'ingrédient pharmaceutique actif est de 10 à 100 µm, la détermination de la taille de particule est effectuée sur le granulomètre laser Malvern MS2000 par voie humide selon les exigences de fonctionnement de l'instrument, le dispersant était une solution aqueuse de Tween 80 et la fraction massique de Tween 80 dans la solution aqueuse était de 0,5 % ;
dans laquelle l'ingrédient pharmaceutique actif est présent en une quantité de 20 à 65 parties en poids ou de 20 à 60 parties en poids, préférablement de 25 à 60 parties en poids, plus préférablement de 40 à 61 parties en poids, et encore plus préférablement de 56 à 61 parties en poids pour 100 parties en poids de la composition pharmaceutique ;
le support comprend une charge ; la charge est un ou plusieurs parmi amidon, amidon prégélatinisé, amidon partiellement prégélatinisé, lactose, saccharose, mannitol, sorbitol, phosphate de calcium hydraté, phosphate de calcium anhydre et cellulose microcristalline, et plus préférablement un ou plusieurs parmi amidon, lactose et cellulose microcristalline ; la charge est préférablement présente en une quantité de 30 à 300 parties en poids, plus préférablement de 50 à 100 parties en poids, encore plus préférablement de 30 à 90 parties en poids, et toujours encore plus préférablement de 30 à 75 parties en poids pour 100 parties en poids de l'ingrédient pharmaceutique actif ;
le support comprend un liant ; le liant est un ou plusieurs parmi hydroxypropylcellulose, hydroxypropylméthylcellulose, polyvinylpyrrolidone et amidon, plus préférablement un ou plusieurs parmi polyvinylpyrrolidone, hydroxypropylméthylcellulose et amidon, et encore plus préférablement polyvinylpyrrolidone et/ou amidon ; le liant est préférablement présent en une quantité de 0 à 100 parties en poids, et non de 0, plus préférablement de 2 à 50 parties en poids, et encore plus préférablement de 30 à 50 parties en poids pour 100 parties en poids de l'ingrédient pharmaceutique actif ;
le support comprend un désintégrant ; le désintégrant est un ou plusieurs parmi hydroxypropylcellulose faiblement substituée, carboxyméthylcellulose calcique, carboxyméthylamidon sodique, croscarmellose sodique et crospovidone, plus préférablement hydroxypropylcellulose faiblement substituée et/ou crospovidone, et encore plus préférablement hydroxypropylcellulose faiblement substituée ; le désintégrant est préférablement présent en une quantité de 0 à 100 parties en poids, et non de 0, plus préférablement de 1 à 25 parties en poids ou de 5 à 25 parties en poids, encore plus préférablement de 1 à 10 parties en poids, de 2 à 10 parties en poids ou de 5 à 10 parties en poids, et toujours encore plus préférablement de 5 à 6 parties en poids pour 100 parties en poids de l'ingrédient pharmaceutique actif ;
le support comprend un lubrifiant ; le lubrifiant est un ou plusieurs parmi stéarate de magnésium, stéarylfumarate de sodium et dodécylsulfate de sodium, et plus préférablement, stéarylfumarate de sodium et/ou dodécylsulfate de sodium ; le lubrifiant est préférablement présent en une quantité de 0,25 à 20 parties en poids, plus préférablement de 0,5 à 5 parties en poids, et encore plus préférablement de 2,5 à 4 parties en poids pour 100 parties en poids de l'ingrédient pharmaceutique actif.

2. Composition pharmaceutique selon la revendication 1, **caractérisée en ce que** la taille de particule D₉₀ de l'ingrédient pharmaceutique actif est de 10 à 70 µm, et préférablement de 40 à 70 µm ;
et/ou, la taille de particule D₅₀ de l'ingrédient pharmaceutique actif est de 4 à 50 µm, préférablement de 4 à 40 µm ou de 4 à 35 µm, et plus préférablement de 10 à 40 µm ;
et/ou, la taille de particule D₁₀ de l'ingrédient pharmaceutique actif est de 0 à 10 µm, et n'est pas de 0, préférablement de 0,1 à 6 µm ou de 2 à 10 µm, et plus préférablement de 2 à 6 µm.

3. Composition pharmaceutique selon la revendication 1 ou 2, **caractérisée en ce que** le support comprend un agent glissant ; l'agent glissant est préférablement un ou plusieurs parmi la silice, le talc et le dodécylsulfate de sodium, plus préférablement la silice et/ou le dodécylsulfate de sodium, et encore plus préférablement le dodécylsulfate de sodium ; l'agent glissant est présent préférablement en une quantité de 0 à 20 parties en poids, et non de 0, plus préférablement de 0,5 à 10 parties en poids, et encore plus préférablement de 4 à 10 parties en poids pour 100 parties en poids de l'ingrédient pharmaceutique actif.

4. Composition pharmaceutique selon la revendication 1 ou 2, **caractérisée en ce que** le support comprend une charge et un liant, les types de charge et de liant étant tels que définis dans la revendication 1, et la charge et le liant étant présents en une quantité totale de 30 à 300 parties en poids, préférablement de 32 à 280 parties en poids, plus préférablement de 32 à 140 parties en poids, et encore plus préférablement de 56,5 à 70 parties en poids pour 100 parties en poids de l'ingrédient pharmaceutique actif ;
et/ou, le support comprend un lubrifiant et un agent glissant, les types de lubrifiant et d'agent glissant étant tels que définis dans la revendication 1, et le lubrifiant et l'agent glissant étant présents en une quantité totale de 1 à 10 parties en poids, préférablement de 1 à 15 parties en poids, plus préférablement de 2,5 à 10 parties en poids, et encore plus préférablement de 2,5 à 4 parties en poids pour 100 parties en poids de l'ingrédient pharmaceutique actif.

5. Composition pharmaceutique selon l'une quelconque des revendications 1 à 4, **caractérisée en ce que** la composition pharmaceutique comprend en outre un agent d'enrobage ; l'agent d'enrobage est préférablement l'hydroxypropylméthylcellulose et/ou le poly(alcool vinylique), et plus préférablement l'hydroxypropylméthylcellulose ; l'agent d'enrobage est préférablement présent dans la composition pharmaceutique en une quantité de 3 % à 15 % en masse, et plus préférablement de 9 % à 13 % en masse par rapport à la masse de la composition pharmaceutique non enrobée.

6. Composition pharmaceutique selon la revendication 1, **caractérisée en ce que** la taille de particule D₅₀ de l'ingrédient pharmaceutique actif est de 4 à 35 µm, la taille de particule D₁₀ de l'ingrédient pharmaceutique actif est de 0,1 à 6 µm ; l'ingrédient pharmaceutique actif est présent en une quantité de 56 à 61 parties en poids pour 100 parties en poids de la composition pharmaceutique ;
le support comprend une charge et un liant, la charge est un ou plusieurs parmi l'amidon, le lactose et la cellulose microcristalline, le liant étant la polyvinylpyrrolidone et/ou l'amidon ; la charge et le liant sont présents en une quantité totale de 56,5 à 70 parties en poids pour 100 parties en poids de l'ingrédient pharmaceutique actif ;
le support comprend un désintégrant, le désintégrant est de l'hydroxypropylcellulose faiblement substituée ; le désintégrant est présent en une quantité de 5 à 6 parties en poids pour 100 parties en poids de l'ingrédient pharmaceutique actif ;
le support comprend l'adjuvant I, l'adjuvant I est le stéarylfumarate de sodium et/ou le dodécylsulfate de sodium ; l'adjuvant I est présent en une quantité de 2,5 à 4 parties en poids pour 100 parties en poids de l'ingrédient pharmaceutique actif ;
la composition pharmaceutique comprend également un agent d'enrobage, l'agent d'enrobage est l'hydroxypropylméthylcellulose, l'agent d'enrobage est présent dans la composition pharmaceutique en une quantité de 9 % à 13 % en masse par rapport à la masse de la composition pharmaceutique non enrobée.

7. Comprimé de nitroxoline, **caractérisé en ce que** le comprimé de nitroxoline est préparé à partir de la composition pharmaceutique selon l'une quelconque des revendications 1 à 6.

8. Procédé de préparation d'un comprimé de nitroxoline, **caractérisé en ce que** la composition pharmaceutique dans le procédé est la composition pharmaceutique selon l'une quelconque des revendications 1 à 6, la composition pharmaceutique comprend l'adjuvant II, l'adjuvant II est un lubrifiant et/ou un agent glissant ;
le procédé comprend les étapes de soumission des composants de la composition pharmaceutique, à l'exception de l'adjuvant II, à une granulation humide, à un broyage humide, à un séchage et à un broyage à sec, l'ajout de l'adjuvant II, la soumission du mélange résultant à un mélange total et la compression pour obtenir le comprimé de nitroxoline ;
lorsque la composition pharmaceutique comprend un agent d'enrobage, l'enrobage est effectué après la compression.

9. Composition pharmaceutique selon l'une quelconque des revendications 1 à 6 et/ou comprimé de nitroxoline selon la revendication 7 pour une utilisation dans le traitement d'un cancer ; préférablement le cancer de la vessie.

10. Composition de comprimé solide de nitroxoline, **caractérisée en ce qu'**elle comprend un ingrédient pharmaceutique actif uniformément dispersé dans celle-ci et un support pharmaceutiquement acceptable, l'ingrédient pharmaceutique actif est la nitroxoline ou un sel pharmaceutiquement acceptable correspondant, la taille de particule D₉₀ de l'ingrédient pharmaceutique actif est de 10 à 100 µm, préférablement de 10 à 70 µm, et plus préférablement de 40 à 70 µm, la détermination de la taille de particule est effectuée sur le granulomètre laser Malvern MS2000 par voie humide conformément aux exigences de fonctionnement de l'instrument, le dispersant était une solution aqueuse de Tween 80 et la fraction massique de Tween 80 dans la solution aqueuse était de 0,5 % ;
l'ingrédient pharmaceutique actif est présent en une quantité de 20 à 60 parties en poids, et préférablement de 25 à 60 parties en poids pour 100 parties en poids de la composition pharmaceutique ;
dans laquelle le support pharmaceutiquement acceptable comprend une charge, un désintégrant, un liant, un agent glissant et un lubrifiant ;
dans laquelle la charge est un ou plusieurs parmi amidon, amidon prégélatinisé, amidon partiellement prégélatinisé, lactose, saccharose, mannitol, sorbitol, phosphate de calcium hydraté, phosphate de calcium anhydre et cellulose microcristalline ; préférablement, la charge est présente en une quantité de 30 à 300 parties en poids, préférablement de 50 à 100 parties en poids, plus préférablement de 30 à 90 parties en poids, et encore plus préférablement de 30 à 75 parties en poids pour 100 parties en poids de l'ingrédient pharmaceutique actif ;
le désintégrant est un ou plusieurs parmi hydroxypropylcellulose faiblement substituée, carboxyméthylcellulose calcique, carboxyméthylamidon sodique, croscarmellose sodique et crospovidone, préférablement, le désintégrant est présent en une quantité de 0 à 100 parties en poids, et non de 0, préférablement de 5 à 25 parties en poids, et plus préférablement de 5 à 10 parties en poids pour 100 parties en poids de l'ingrédient pharmaceutique actif ;
le liant est un ou plusieurs parmi hydroxypropylcellulose, hydroxypropylméthylcellulose, polyvinylpyrrolidone et amidon ; préférablement, le liant est présent en une quantité de 0 à 100 parties en poids, et non de 0, préférablement de 2 à 50 parties en poids, et plus préférablement de 30 à 50 parties en poids pour 100 parties en poids de l'ingrédient pharmaceutique actif ;
le lubrifiant est un ou plusieurs parmi stéarate de magnésium, stéarylfumarate de sodium et dodécylsulfate de sodium ; préférablement, le lubrifiant est présent en une quantité de 0,25 à 20 parties en poids, préférablement de 1 à 5 parties en poids, et plus préférablement de 2,5 à 4 parties en poids pour 100 parties en poids de l'ingrédient pharmaceutique actif.

11. Composition de comprimé solide à base de nitroxoline selon la revendication 10, **caractérisée en ce que** la taille de particule D₅₀ de l'ingrédient pharmaceutique actif est de 4 à 50 µm, préférablement de 4 à 40 µm, et plus préférablement de 10 à 40 µm ;
et/ou, la taille de particule D₁₀ de l'ingrédient pharmaceutique actif est de 0 à 10 µm, et non de 0, préférablement de 2 à 10 µm, et plus préférablement de 2 à 6 µm.

12. Composition de comprimé solide à base de nitroxoline selon la revendication 10 ou 11, **caractérisée en ce que** le support pharmaceutiquement acceptable comprend en outre un ou plusieurs parmi un agent glissant, une matière colorante, un agent d'ajustement du pH, un tensioactif, un stabilisant et une fragrance.

13. Composition de comprimé solide de nitroxoline selon la revendication 10 ou 11, **caractérisée en ce que**
le support pharmaceutiquement acceptable comprend en outre l'agent glissant, lequel l'agent glissant est un ou plusieurs parmi la silice, le talc et le dodécylsulfate de sodium ; préférablement, l'agent glissant est présent en une quantité de 0 à 20 parties en poids, et non de 0, préférablement de 0,5 à 10 parties en poids, et plus préférablement de 4 à 10 parties en poids pour 100 parties en poids de l'ingrédient pharmaceutique actif.

14. Composition de comprimé solide de nitroxoline selon l'une quelconque des revendications 10 à 13, **caractérisée en ce que** la composition de comprimé solide de nitroxoline comprend en outre un matériau d'enrobage pelliculaire ;
le matériau d'enrobage pelliculaire est préférablement de l'hydroxypropylméthylcellulose et/ou du poly(alcool vinylique) ;
le gain pondéral du matériau d'enrobage pelliculaire est préférablement de 3 % à 15 % pour 100 parties en poids de l'ingrédient pharmaceutique actif.

15. Comprimé de nitroxoline selon la revendication 7, **caractérisé en ce qu'**il comprend :
nitroxoline 100 parties en poids,
charge 30 à 300 parties en poids, préférablement 50 à 100 parties en poids,
désintégrant 0 à 100 parties en poids, préférablement 5 à 25 ou 5 à 10 parties en poids,
liant 0 à 100 parties en poids, préférablement 5 à 25 ou 2 à 50 parties en poids,
lubrifiant 0,25 à 20 parties en poids, préférablement 2,5 à 4 ou 0,5 à 5 parties en poids,
agent glissant 0 à 20 parties en poids, préférablement 4 à 10 ou 0,5 à 10 parties en poids, et
poudre d'enrobage pelliculaire facultative 3 % à 15 % de gain de poids pour 100 parties en poids de nitroxoline, et
la taille de particule D₉₀ de la nitroxoline est de 10 à 100 µm, préférablement de 10 à 70 µm, et plus préférablement de 40 à 70 µm, la détermination de la taille de particule est effectuée sur le granulomètre laser Malvern MS2000 par voie humide selon les exigences de fonctionnement de l'instrument, le dispersant était une solution aqueuse de Tween 80 et la fraction massique de Tween 80 dans la solution aqueuse était de 0,5 % ;
les types de charge, désintégrant, liant, lubrifiant et agent glissant sont tels que définis dans la revendication 10.
